# EUROPEAN PATENT APPLICATION

(11) **EP 1 555 001 A1**
(43) Date of publication of application: **20.07.2005**
(21) Application number: 04292810.1
(22) Date of filing: 29.11.2004
(51) Int. Cl.: A61F 13/38

(54) **A swab for removal of water inside the ears**

(30) Priority: 14.01.2004 KR 2004000974
(71) Applicant: Cynn, Chi-Ho, Pochun-city 487-804 Kyungki-do (KR)
(72) Inventor: Cynn, Chi-Ho, Pochun-city 487-804 Kyungki-do (KR)
(74) Representative: Schrimpf, Robert

(57) **Abstract**

A swab for removal of water inside the ears adapted to effectively and painlessly remove the water remaining in the external auditory meatus after swim or bath that might cause discomfort and auditory inflammation, integrally comprises a long conical shape, which is wholly made of high water-absorbing material. One end is a thick handle part which has appropriate solidity, the other end is a thin pointed part which is very soft and supple. The thick handle part is adequately solid to grip with fingers and as it goes to the thin pointed part, it becomes gradually soft and supple.

## Description

### FIELD OF THE INVENTION

The present invention relates to a swab for removal of water inside the ears. More particularly, it is adapted to remove water effectively, painlessly and safely which remains in the external auditory meatus after swim or bath that might cause discomfort and auditory inflammation.

### BACKGROUND OF THE INVENTION

Generally, water often comes into the ear during swim or bath even though we pay attention and it gives rise to discomfort or various diseases such as tympanitis unless it is quickly removed.

Usually we use common swabs to remove water inside the ears. But we cannot get the purpose as easily as we expected. Because the common swab cannot be inserted inside the ears deeply enough to remove the water effectively. And even if we try to insert the plain swab forcibly into the ears, we just can find it impossible. It causes pain only.

### SUMMARY OF THE INVENTION

The present invention is to solve the aforementioned problems. So it is the important ability for the present invention to remove the water inside the ears effectively, quickly, even more safely. If it is possible, we can enjoy swimming or bathing without worry.

In accordance with the preferred embodiment of the present invention, this swab integrally comprises: a long conical shape, which is made of high water-absorbing material such as tissue paper, compressed cotton or compressed pulp, etc. One end of the conical shape is a thick handle part having appropriate solidity. And the other end is a thin pointed tip which is very soft and supple.

### BRIEF DESCRIPTION OF THE DRAWINGS

To understand the character of the present invention more easily, drawings and brief descriptions will be necessary.

FIG 1 is a perspective view of the swab according to the embodiment of the present invention.

FIG 2 is an example drawing to illustrate the constitution using fanwise tissue paper.

FIG 3 is an example drawing to illustrate the swab which is made of compressed cotton or compressed pulp.

FIG 4 is an example drawing to illustrate how the present invention is applied in actual use.

### DETAILED DESCRIPTION OF THE PREFERRED EMBODIMENT

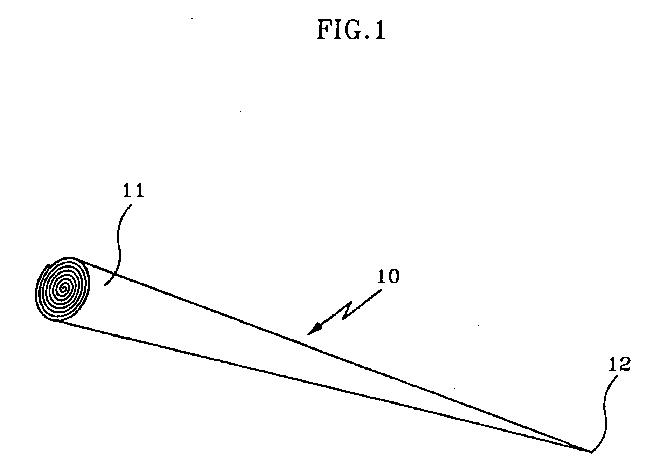

As we can see in FIGS. 1, 2, 3, and 4, a swab 10 which is designed to remove the water inside the ears integrally includes a handle part 11 that has appropriate solidity and a pointed end part 12 which is very soft and supple.

The swab 10 has a long conical shape which is wholly made of high water-absorbing materials such as tissue paper, compressed cotton, compressed pulp etc. So it can absorb the water inside the ears thoroughly, quickly and effectively.

The thick handle part 11 is solid enough to grip with fingers and it becomes gradually thin and soft and supple as it goes to the other thin pointed end part 12. So the swab is very flexible and easily inserted inside the ear without any pain or wounds.

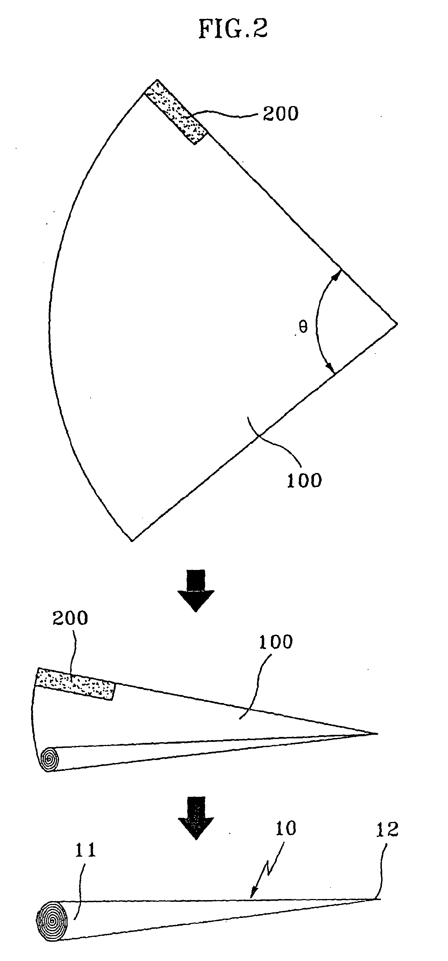

In reference to the embodiment of the present invention as depicted in FIGS. 1 and 2, the swab 10 is preferably made of rolled fanwise tissue paper 100. It is advisable for the fanwise tissue paper to have a center angle (θ) of 90 degrees as shown in FIG 2.

If necessary, the rim of the tissue paper 100 can be partly coated with adhesive 200 to prevent unfastening or unraveling as illustrated in FIG 2. When the rim of the tissue paper 100 is coated with adhesive 200, the rolled paper is good enough to maintain the long conical shape.

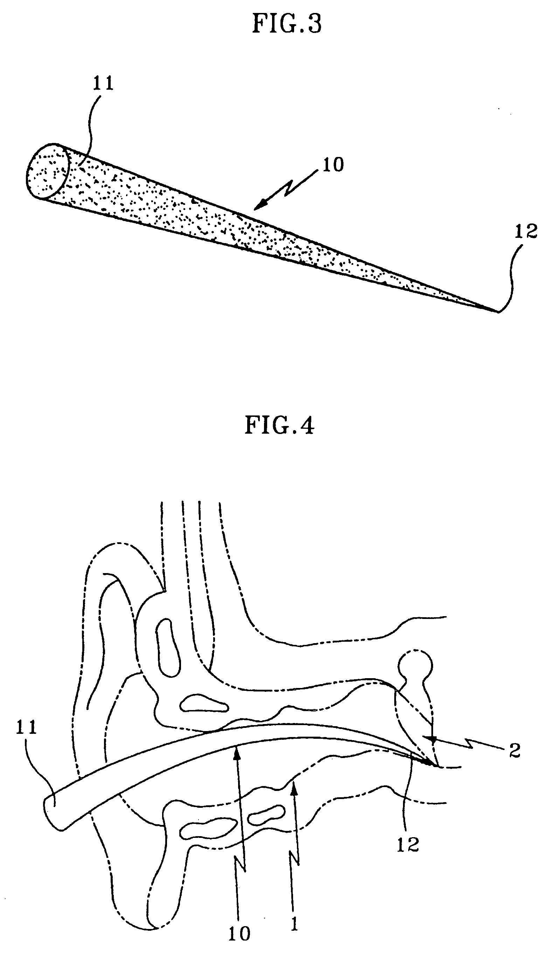

In FIG 3, the swab is made of compressed cotton or compressed pulp which can absorb the water inside the ear as well.

The process of removing the water by using the swab 10 is shown in FIG 4.

When the pointed end 12 is slowly inserted into the ear, the pointed end 12 is thin, very soft and supple, and it becomes gradually thick and solid as it goes to the handle part 11, it is possible to be bent easily along with the way of the external auditory meatus.

And the pointed end 12 can be dipped in the water directly. As soon as the pointed end 12 meets the water, it begins to absorb the water which remains around the ear drum 2 according to the capillary action.

It takes about 10 seconds for the swab 10 to absorb and remove the water thoroughly.

Even though the pointed end 12 touches the wall of the external auditory meatus 1 or the ear drum 2 when it is inserted into the ear, it does not cause any harm, pain or wounds. Because the tip is very soft and supple.

Moreover, the swab 10 can be used for the children because it is very safe.

And there is another advantage in that we can prevent the ear diseases beforehand which can be caused by the remaining water inside the ears like tympanitis.

The preferred embodiments of the present invention do not limit the scope of the invention. It is not intended to limit the precise form as it is disclosed.

Modifications and variations are possible in light of the above teachings.

It is intended that the scope of the invention is to be defined by the claims appended hereto, and their equivalents.

## Claims

1. A swab for removal of water inside the ears integrally comprising:
a long conical shape, which is wholly made of high water-absorbing material;
one end being a thick handle part which has appropriate solidity, the other end being a thin pointed part which is very soft and supple;
wherein the thick handle part is adequately solid to grip with fingers and as it goes to the thin pointed part, it becomes gradually soft and supple.

2. The swab, as defined in claim 1, made of rolled fanwise tissue paper.

3. A rim of the tissue paper, as defined in claim 2, partly coated with adhesive to prevent unfastening or unraveling.

4. The swab, as defined in claim 1, comprised with high water-absorbing materials such as compressed cotton, compressed pulp, etc.
